# EUROPÄISCHE PATENTANMELDUNG

(11) **EP 1 452 154 A2**
(43) Veröffentlichungstag der Anmeldung: **01.09.2004**
(21) Anmeldenummer: 04003130.4
(22) Anmeldetag: 12.02.2004
(51) Int. Cl.: A61F 5/01, A61F 5/058

(54) **Orthese mit Halteband**

(30) Priorität: 15.02.2003 DE 20302451 U
(71) Anmelder: Thämert Orthopädische Hilfsmittel GmbH & Co. KG, 30938 Burgwedel/Grossburgwedel (DE)
(72) Erfinder: Kiefer, Roland, 77855 Achern (DE)
(74) Vertreter: Siekmann, Gunnar, Dipl.-Phys.

(57) **Zusammenfassung**

Bei einer Orthese (1) mit mindestens einem Halteband (3,4), das zum Anlegen an den Körper eines Patienten, insbesondere an Arme oder Beine eines Patienten, bestimmt ist, weist das Halteband (3,4) eine vorgespannte, profilierte Metallschiene auf, die so ausgelegt ist, daß das Halteband (3,4) der Vorspannung der Metallschiene folgend aus einer ersten gestreckten Position in eine zweite, am Körper des Patienten anliegende Position wechseln kann. Dadurch läßt sich die Orthese (1) besonders einfach anlegen.

## Beschreibung

Die Erfindung betrifft eine Orthese mit mindestens einem Halteband, das zum Anlegen an den Körper eines Patienten, insbesondere an Arme oder Beine eines Patienten, bestimmt ist.

Ein Beispiel einer solchen Orthese ist die in der EP 0 916 325 A1 beschriebene Karpaltunnelorthese. Beim Karpaltunnelsyndrom kommt es aufgrund einer Nerveneinklemmung im Handwurzelbereich zu Gefühlsstörungen und Schmerzen des Daumens, des Zeigefingers und des Mittelfingers der betroffenen Hand. Eine mögliche Therapie ist die Ruhigstellung des Handgelenkes mit einer Schiene, einer sogenannten Karpaltunnelorthese, so wie sie in der genannten Druckschrift beschrieben wird. Diese, wie auch andere Orthesen werden mit Hilfe von Haltebändern am Körper, hier am Handgelenk und am Handteller, fixiert. Dabei werden entweder starre vorgebogene oder biegbare Haltebänder und/oder Halteeinrichtungen mit Kunststoff- und/oder Metalleinsätzen oder Haltebänder aus textilen Materialien eingesetzt, die sich in der Regel mit Klettverschlüssen miteinander verbinden lassen.

Der Erfindung liegt die Aufgabe zugrunde, eine Orthese der eingangs genannten Art zu schaffen, die sich besonders einfach anlegen läßt.

Die Lösung dieser Aufgabe erfolgt mit einer Orthese mit den Merkmalen des Patentanspruchs 1. Vorteilhafte Weiterbildungen der Erfindung sind in den Unteransprüchen angegeben.

Erfindungswesentlich ist, daß das Halteband der Orthese eine vorgespannte, profilierte Metallschiene aufweist, die so ausgelegt ist, daß das Halteband der Vorspannung der Metallschiene folgend aus einer ersten gestreckten Position in eine zweite am Körper des Patienten anliegende Position wechseln kann.

Die gestreckte Form wird im wesentlichen dadurch erreicht bzw. gehalten, daß die in dem Halteband angeordnete Metallschiene in der durch das Profil vorgegebenen Position gehalten ist. Bei Ausübung eines leichten Drucks auf die Metallschiene, insbesondere in den Endbereichen, verläßt die Metallschiene ihre profilierte Form und kann dann der Vorspannung folgen, die entgegengesetzt zu dem Profil ausgerichtet ist, und sich ring- oder kreisartig an den Körper des Patienten, insbesondere an einen Arm oder ein Bein, anlegen.

Die vorgespannte, profilierte Metallschiene ist bevorzugt aus Federstahl, hartgewalztem Aluminium, Berylliumbronze oder einem vergleichbaren Material hergestellt. Wichtig ist einerseits, daß sich die Metallschiene vorspannen läßt und andererseits, daß ein Profil einbringbar ist, wobei das Profil und die Vorspannung in entgegengesetzte Richtungen ausgebildet sind. Die Metallschiene ist bevorzugt derart vorgespannt, daß sie sich der Vorspannung folgend ringförmig aufdreht. Dabei muß es sich nicht um einen vollständigen oder geschlossenen Ring handeln, sondern bevorzugt ist die Metallschiene so lang ausgebildet, daß sie das zu umfassende Gliedmaß zu etwa 2/3 umgreift. In einer bevorzugten Weiterbildung der Erfindung wird das Halteband zusammen mit einem an dem Halteband befestigbaren Gurt verwendet, so daß das Halteband zusammen mit dem über Klettverschlüsse daran befestigbaren Gurt zu einem geschlossenen Ring schließbar ist, der sich um einen Arm oder ein Bein des Patienten schließt.

Die Metallschiene ist mindestens auf der dem Patienten zugewandten Seite mit einem weichen Material, insbesondere einem elastomeren Material, einem Schaumstoff oder auch einem textilen Material, bedeckt. Bevorzugt ist die Metallschiene beweglich in einer geschlossenen Tasche eines die Metallschiene umgebenden Materials angeordnet. Dies kann ein elastomeres, ein schaumstoffartiges oder auch ein textiles Material sein. Durch die Beweglichkeit innerhalb dieser Tasche ist gewährleistet, daß die Vorspannung der Metallschiene beim Anlegen der Orthese zum Einsatz kommt ohne durch mit der Metallschiene verklebte Auflagen behindert zu werden.

In einer besonders bevorzugten Ausgestaltung der Erfindung ist die Orthese als Karpaltunnelorthese ausgebildet. Die Karpaltunnelorthese zeichnet sich insbesondere durch zwei teilkreisförmige Haltebänder mit vorgespannten, profilierten Metallschienen zur flexiblen Anformung an einen Unterarm eines Patienten aus. Die Haltebänder sind bevorzugt an einer starren Schiene angeordnet, die zur Anlage an der Arminnenseite vorgesehen ist. Es ergibt sich dadurch eine Kreuzform mit zweitem Querbalken oder auch die Form eines großen T's, bei dem in der Mitte ein zusätzlicher Querbalken vorgesehen ist. Dabei ist die Mittelschiene starr, während die beiden Haltebänder zunächst in der gestreckten Stellung sind, jedoch bei Verlassen der Profilform sich der Vorspannung der Metallschienen folgend flexibel an den Unterarm des Patienten anlegen. Die starre Schiene weist an einem Endbereich ein erstes quer zur Schiene verlaufendes Halteband, etwa in der Mitte ein zweites quer zur Schiene verlaufendes Halteband und im zweiten Endbereich eine ergänzende Befestigungsmöglichkeit für ein um den Handteller verlaufendes flexibles Halteband auf. Mit Hilfe dieser drei Haltebänder wird das Handgelenk ruhiggestellt.

Bevorzugt ist die starre Schiene im Armbereich im wesentlichen gerade und im handseitigen Endbereich mit einer sich in den Handteller hinein erstreckenden Wölbung ausgebildet. Die starre Schiene ist außerdem bevorzugt im handseitigen Endbereich kreisförmig flächig erweitert. Es ist also eine in den Handteller hinein gewölbte flächige Erweiterung vorgesehen, auf der sich die Hand des Patienten abstützt und die ein Abknicken oder Anwinkeln der Hand nach innen verhindert.

Nachfolgend wird die Erfindung anhand eines in der Zeichnung dargestellten bevorzugten Ausführungsbeispiels weiter erläutert. Im einzelnen zeigen die schematischen Darstellungen in:
- Fig. 1:: eine Draufsicht auf eine erfindungsgemäße Karpaltunnelorthese mit den Haltebändern in gestreckter Position;
- Fig. 2:: eine erfindungsgemäße Karpaltunnelorthese mit angebogenen Haltebändern;
- Fig. 3:: eine angelegte Karpaltunnelorthese in einer ersten Ansicht;
- Fig. 4:: die Karpaltunnelorthese gemäß Fig. 3 in einer Ansicht von der Arminnenseite;
und
- Fig. 5:: eine Ansicht einer Metallschiene zur Verwendung in der erfindungsgemäßen Orthese.

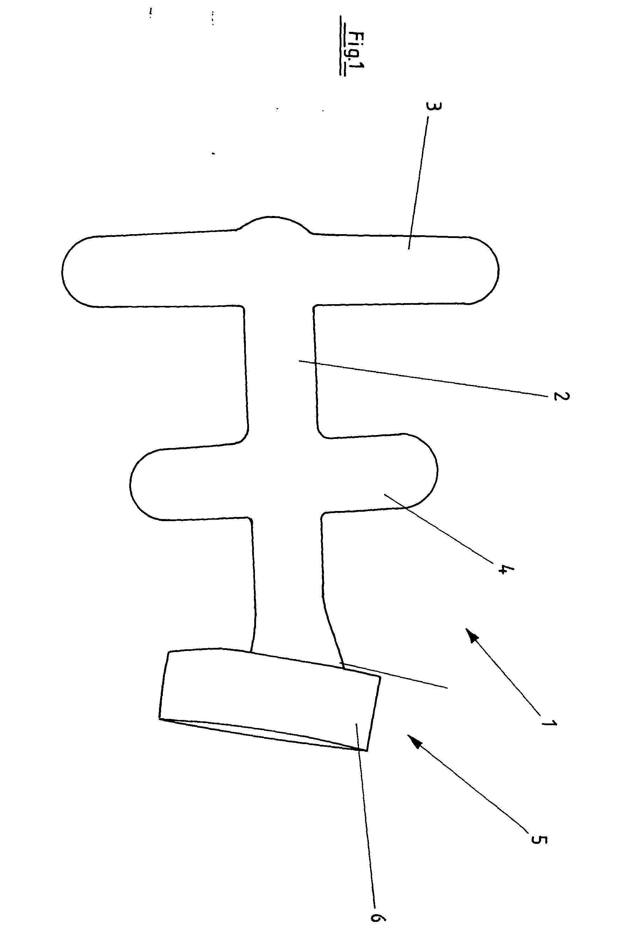

In Fig. 1 ist eine Orthese 1, hier eine Karpaltunnelorthese, dargestellt. Die Karpaltunnelorthese weist eine zentrale starre Schiene 2 auf, an der zwei Haltebänder 3 und 4 senkrecht zur Schiene 2 angeordnet sind. Die Haltebänder 3 und 4 dienen der Anlage und Befestigung der Orthese 1 am Unterarm eines Patienten. Im handseitigen Endbereich 5 der Schiene 2 ist ein flexibles Halteband 6 vorgesehen, das um den Handteller des Patienten gelegt wird. Im Endbereich 5 der Schiene 2 ist die Schiene 2 nach oben gewölbt und erweitert sich flächig in einen kreisförmigen Endbereich 11, der ansatzweise zu sehen ist, jedoch teilweise durch das Halteband 6 verdeckt ist. Dieser kreisförmige Endbereich ist in den Handteller hinein hochgewölbt, so daß sich eine in den Handteller hineingewölbte Auflagefläche ergibt, auf die die Hand abgestützt ist und die ein Abknicken der Hand verhindert. Die starre Schiene 2 und die Haltebänder 3 und 4 weisen auf ihren Außenseiten durchgängig ein elastomeres Material auf. Im Inneren der starren Schiene 2 ist ein starres Metall- oder Kunststoffelement vorgesehen, und in den Haltebändern 3 und 4 sind im Inneren Metallschienen vorgesehen, die eine Vorspannung aufweisen, die ein ringartiges Aufrollen der Haltebänder zur Mitte hin bewirken. Da die Metallschienen in den Haltebändern 3 und 4 profiliert sind, ist es möglich, diese in die in Fig. 1 dargestellte Position zu bringen.

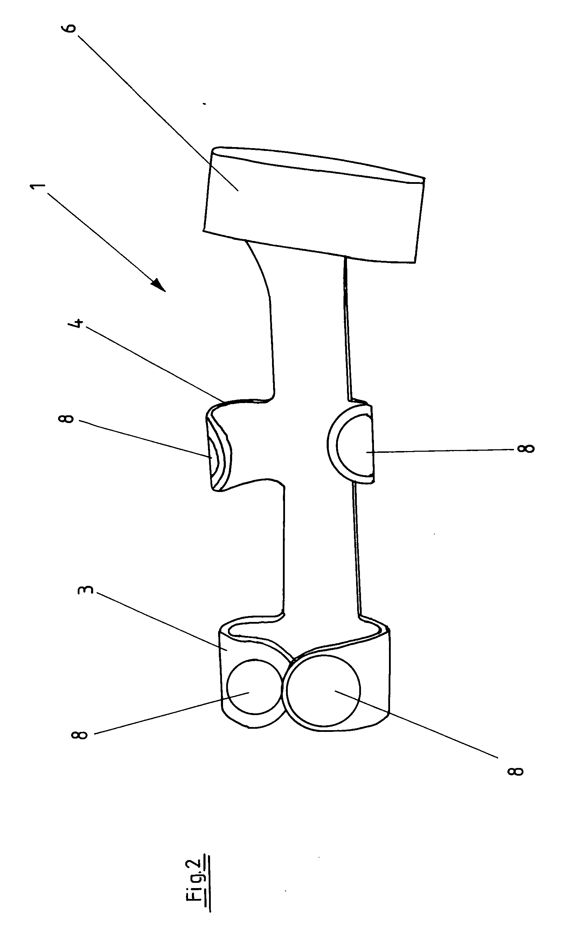

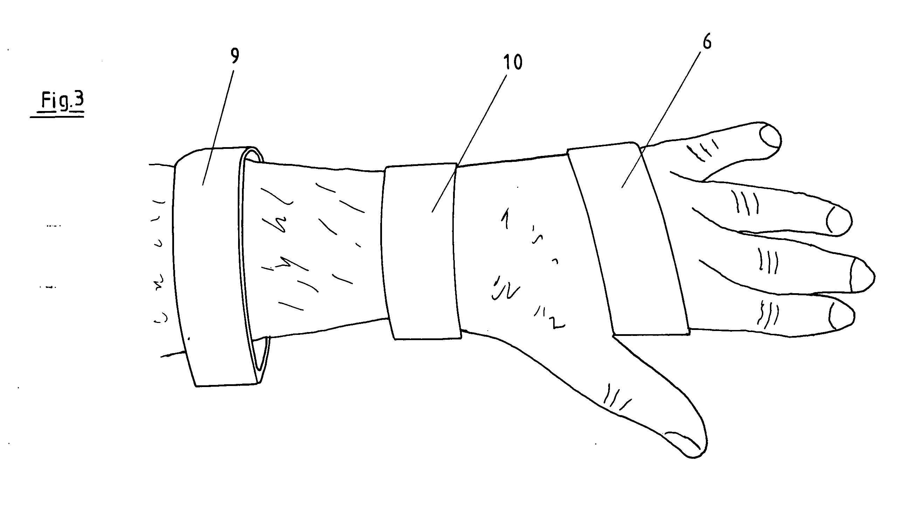

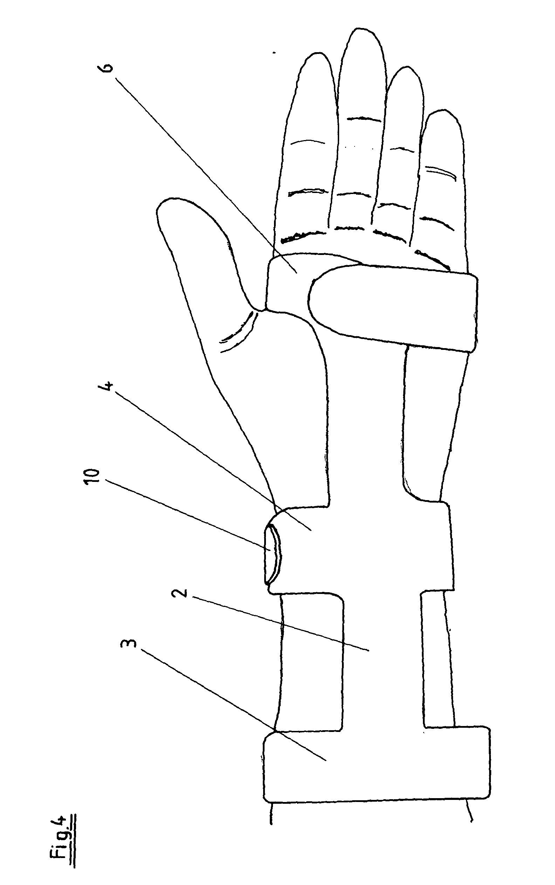

In Fig. 2 ist die Orthese 1 mit angebogenen Haltebändern 3 und 4 dargestellt. An den Endbereichen der Haltebänder 3 und 4 sind Haftelemente 8 vorgesehen, an denen in den Figuren 3 und 4 dargestellte Gurte 9 und 10 befestigbar sind, insbesondere mit Hilfe von Klettverbindungen befestigbar sind, so daß die Haltebänder zusammen mit den Gurten einen um einen Arm oder ein anderes Gliedmaß eines Patienten geschlossenen Ring bilden können.

In Fig. 3 ist eine Draufsicht auf eine angelegte Orthese dargestellt, bei der insbesondere im Handbereich Gurte 9 und 10 dargestellt sind, wobei der Gurt 9 an den Haftelementen 8 des Haltebandes 3 und der Gurt 10 an den Haftelementen 8 des Haltebandes 4 befestigt ist, so daß auf diese Weise jeweils ein geschlossener Ring gebildet wird. Das Halteband 6, das an dem handseitigen Endbereich der Orthese 1 befestigt ist, verläuft um den Handteller oberhalb des Daumens.

In Fig. 4 ist die Arminnenseite mit der angelegten Orthese dargestellt. Hier wird deutlich, daß die starre Schiene 2 auf der Arminnenseite angeordnet ist, wobei das handseitige Ende der Schiene 2 nach oben in die Handfläche hineingewölbt ist.

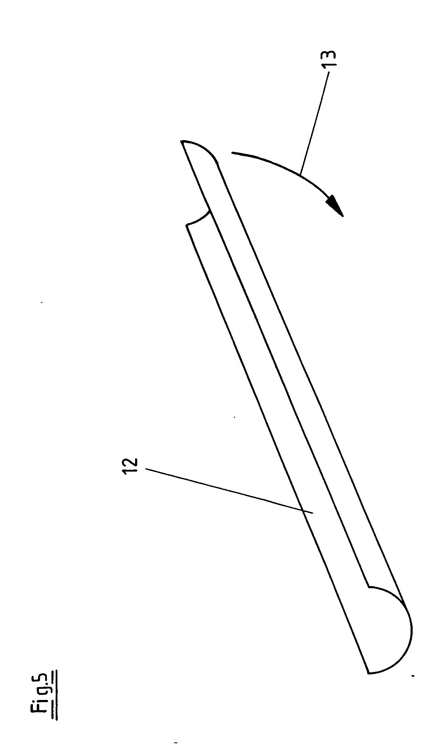

In Fig. 5 ist eine perspektivische Ansicht einer Metallschiene 12 dargestellt, die in eines der Haltebänder 3 und 4 eingelegt ist. Die Metallschiene 2 weist dabei ein Profil bzw. eine Wölbung auf, die in der Figur eine nach oben offene Rinne bildet. Durch diese Profilierung der Metallschiene 12 ist die Metallschiene 12 in der gezeigten Position stabil. Durch Ausüben von leichtem Druck auf die Metallschiene 12, bei der die Schiene aus der Profilform herausgebogen wird, verliert das Profil seine stabilisierende Wirkung, und die Metallschiene wird sich dann entsprechend seiner Vorspannung in Richtung des Pfeils 13 aufrollen.

## Patentansprüche

1. Orthese mit mindestens einem Halteband (3, 4), das zum Anlegen an den Körper eines Patienten, insbesondere an Arme oder Beine eines Patienten, bestimmt ist,
**dadurch gekennzeichnet,**
**daß** das Halteband (3, 4) eine vorgespannte, profilierte Metallschiene (12) aufweist, die so ausgelegt ist, daß das Halteband (3, 4) der Vorspannung der Metallschiene (12) folgend aus einer ersten gestreckten Position in eine zweite, am Körper des Patienten anliegende Position wechseln kann.

2. Orthese nach Anspruch 1, **dadurch gekennzeichnet, daß** die vorgespannte profilierte Metallschiene (12) aus Federstahl, hartgewalztem Aluminium oder Berylliumbronze hergestellt ist.

3. Orthese nach einem der Ansprüche 1 oder 2, **dadurch gekennzeichnet, daß** die Metallschiene (12) derart vorgespannt ist, daß sie sich der Vorspannung folgend ringartig aufdreht.

4. Orthese nach einem der vorhergehenden Ansprüche, **dadurch gekennzeichnet, daß** die Metallschiene (12) mindestens auf der dem Patienten zugewandten Seite ein elastomeres Material aufweist.

5. Orthese nach einem der vorhergehenden Ansprüche, **dadurch gekennzeichnet, daß** die Metallschiene (12) beweglich in einer geschlossenen Tasche eines die Metallschiene (12) umgebenden Materials angeordnet ist.

6. Orthese nach einem der vorhergehenden Ansprüche, **dadurch gekennzeichnet, daß** das Halteband (3, 4) im angelegten Zustand einen Teilkreis bildet.

7. Orthese nach einem der vorhergehenden Ansprüche, **dadurch gekennzeichnet, daß** das Halteband (3, 4) zusammen mit einem an dem Halteband (3, 4) befestigbaren Gurt (9, 10) zu einem Ring schließbar ist.

8. Orthese nach einem der vorhergehenden Ansprüche, **dadurch gekennzeichnet, daß** die Orthese (1) als Karpaltunnelorthese ausgebildet ist.

9. Orthese nach Anspruch 8, **dadurch gekennzeichnet, daß** die Karpaltunnelorthese zwei teilkreisförmige Haltebänder (3, 4) mit vorgespannten profilierten Metallschienen (12) zur flexiblen Anformung an einen Unterarm eines Patienten aufweist.

10. Orthese nach einem der Ansprüche 8 oder 9, **dadurch gekennzeichnet, daß** die Haltebänder (3, 4) an einer starren Schiene (2) angeordnet sind, die zur Anlage an der Arminnenseite vorgesehen ist.

11. Orthese nach einem der Ansprüche 8 bis 10, **dadurch gekennzeichnet, daß** die starre Schiene (2) an einem Endbereich ein erstes quer zur Schiene (2) verlaufendes Halteband (3) und etwa in der Mitte ein zweites quer zur Schiene (2) verlaufendes Halteband (4) aufweist und im zweiten Endbereich eine Befestigungsmöglichkeit für ein um den Handteller laufendes flexibles Halteband (6) vorgesehen ist.

12. Orthese nach einem der Ansprüche 8 bis 11, **dadurch gekennzeichnet, daß** die starre Schiene (2) im Armbereich im wesentlichen gerade ist und im handseitigen Endbereich (5) eine sich in den Handteller erstreckende Wölbung aufweist.

13. Orthese nach einem der Ansprüche 8 bis 12, **dadurch gekennzeichnet, daß** die starre Schiene im handseitigen Endbereich (5) kreisförmig flächig erweitert ist.
